# EUROPEAN PATENT APPLICATION

(11) **EP 4 756 828 A1**
(43) Date of publication of application: **10.06.2026**
(21) Application number: 23948578.2
(22) Date of filing: 14.08.2023
(51) Int. Cl.: G16H 20/70, G16H 10/60, G16H 50/70, G16H 50/50

(54) **METHOD FOR DEVELOPING DIGITAL THERAPEUTICS THROUGH MODULAR DIGITAL THERAPEUTIC FRAMEWORK AND APPARATUS USING SAID METHOD**

(30) Priority: 04.08.2023 KR 20230102210
(71) Applicant: WELT Corp., Ltd, Seocho-gu Seoul 06628 (KR)
(72) Inventor: KANG, Seong Ji, Seoul 06366 (KR); ROH, Hye Kang, Seoul 05507 (KR); KIM, Joo Young, Seoul 04014 (KR); LEE, Do Hyun, Yongin-si Gyeonggi-do 16826 (KR); JEONG, Hwa Young, Incheon 21451 (KR)
(74) Representative: AWA Sweden AB
(86) International application number: PCT/KR2023/012019
(87) International publication number: WO 2025/033583

(57) **Abstract**

The present invention relates to a method for developing digital therapeutics through a modular digital therapeutic framework and an apparatus using the method. The method for developing digital therapeutics through a modular digital therapeutic framework may comprise the steps of: a digital therapeutics generation unit receiving digital therapeutics data; and the digital therapeutics generation unit generating digital therapeutics on the basis of the digital therapeutics data

## Description

### BACKGROUND

### 1. Field of the Invention

The present invention relates to a method of developing a digital therapy solution through a modular digital therapy framework and an apparatus using the method. More specifically, the present invention relates to a method of developing a digital therapy solution through a modular digital therapy framework for rapidly and easily developing a digital therapy solution and an apparatus using the method.

### 2. Discussion of Related Art

With the development of various smart technologies, data of personal daily activities is recorded, and individual life can be efficiently managed on the basis of the recorded data. In the meantime, health-related data logging is attracting attention due to the increasing interest in healthcare. Many users have already been generating and utilizing various health-related data including data on exercise, diet, sleep, and the like through user devices such as smartphones, wearable devices, and the like. In the past, health-related data was generated and managed only by medical institutions, but now users have begun to generate and manage their own health-related data through user devices such as smartphones and wearable devices.

In many cases, health-related data logging is performed through a wearable device. A wearable device is a user device that is carried by or attached to a user. Due to the development of Internet of things (IoT) and the like, wearable devices are frequently used for collecting health-related data. A wearable device may collect a user's physical change information and surrounding data of the user through equipment and provide advice required for the user's healthcare on the basis of the collected data.

A user's health-related data may include a user biomarker, and research is ongoing on a method of making a medical prescription adaptively to a user on the basis of the user's health-related data.

The technology was developed through the 'R&D for Digital Healthcare Demonstration and Adoption in Medical Institutions in 2023 (RS-2023-00266002) "Multi-Center Clinical Validation and RWE Generation for Insurance Coverage of a Digital Therapeutic for Insomnia" by the Korea Health Industry Development Institute.

As related art, there is Korean Patent No. 10-2425479.

### SUMMARY OF THE INVENTION

The present invention is directed to effectively generating a digital therapy solution by combining different modules through a modular digital therapy framework.

In addition, the present invention is directed to providing a platform for easily generating a new digital therapy solution by combining a digital therapy module, a therapy algorithm, and an artificial intelligence (AI) model that have already been developed.

According to an aspect of the present invention, there is provided a method of developing a therapy solution through a modular digital therapy framework, the method comprises receiving, by a digital therapy solution generator, digital therapy solution data; and generating, by the digital therapy solution generator, a digital therapy solution based on the digital therapy solution data.

Meanwhile, the digital therapy solution data includes data regarding a digital therapy module, a therapy algorithm, or an artificial intelligence (AI) model.

Further, the digital therapy solution is generated based on a combination of the digital therapy module, the therapy algorithm, or the AI model.

According to another aspect of the present invention, there is provided a system for generating a digital therapy solution, which is a system for developing a digital therapy solution through a modular digital therapy framework, the system comprising a digital therapy solution generator configured to receive digital therapy solution data and generate a digital therapy solution based on the digital therapy solution data.

Meanwhile, the digital therapy solution data includes data regarding a digital therapy module, a therapy algorithm, or an artificial intelligence (AI) model.

Further, the digital therapy solution is generated based on a combination of the digital therapy module, the therapy algorithm, or the AI model.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a conceptual diagram illustrating a system for generating a digital therapy solution according to an embodiment of the present invention.
FIG. 2 is a conceptual diagram illustrating the operation of a digital therapy solution generator according to an embodiment of the present invention.
FIG. 3 is a conceptual diagram illustrating the operation of the digital therapy solution generator according to an embodiment of the present invention.
FIG. 4 is a conceptual diagram illustrating a method of developing a digital therapy solution according to an embodiment of the present invention.
FIG. 5 is a conceptual diagram illustrating a method of standardizing digital therapy configurators for connection of digital therapy configurators according to an embodiment of the present invention.
FIG. 6 is a conceptual diagram illustrating a connection relationship between digital therapy configurators according to an embodiment of the present invention.

### DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope and spirit of the present invention. In addition, it should be understood that a position or arrangement of each component in each disclosed embodiment may be changed without departing from the scope and spirit of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

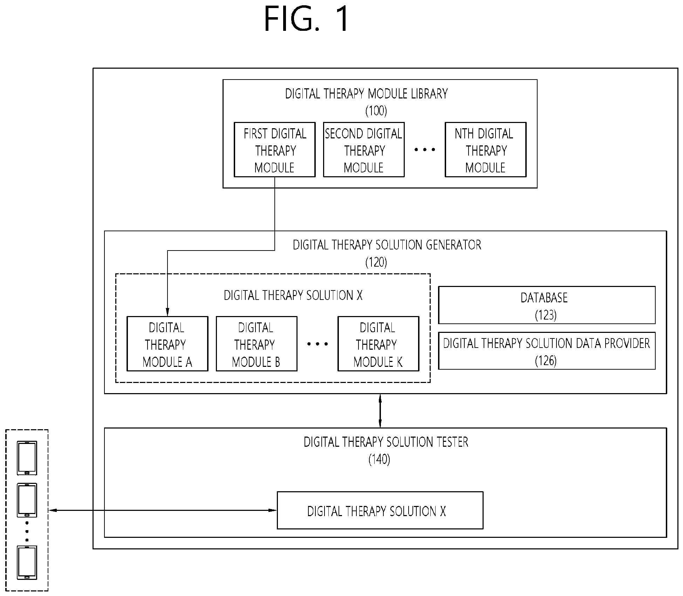

FIG. 1 is a conceptual diagram illustrating a system for generating a digital therapy solution according to an embodiment of the present invention.

In FIG. 1, a system for generating a digital therapy solution for providing a digital therapy solution generating framework for rapidly and easily generating a digital therapy solution is disclosed.

Referring to FIG. 1, the system for generating a digital therapy solution may include a digital therapy module library 100, a digital therapy solution generator 120, and a digital therapy solution tester 140.

The digital therapy module library 100, the digital therapy solution generator 120, and the digital therapy solution tester 140 may be individual hardware devices or an integrated hardware device.

The digital therapy module library 100 may be implemented to provide a digital therapy module that may be used for digital therapy. The digital therapy module may be a module usable in a plurality of different digital therapy applications. For example, the digital therapy module may be a module that may be utilized for digital therapy services on various digital therapy applications, such as a sleep time check module, a dietary habit check module, a heartbeat check module, and the like.

The digital therapy solution generator 120 may be implemented to generate a digital therapy solution based on a digital therapy module. The digital therapy solution may be generated as a combination of various other separately developed modules as well as those in the digital therapy module library 100.

The digital therapy solution generator 120 may include a database 123 for generating a digital therapy solution, a digital therapy solution data provider 126, and the like.

The database 123 may include data regarding diseases. For example, in the case of a migraine, information about patient data and patient therapy data required for generating a digital therapy solution, such as biometric data of migraine patients and therapy data on migraine patients, may be included in the database 123.

In addition, the database 123 may include previous digital therapy history data accumulated through previous digital therapy procedures, and the previous digital therapy history data may be provided to the digital therapy solution generator 120.

The digital therapy solution data provider 126 may provide data regarding a digital therapy module, a digital therapy algorithm, and an artificial intelligence (AI) engine for digital therapy, which constitute the existing digital therapy solution, as digital therapy solution data. The digital therapy module data may include data regarding a module for managing and/or analyzing diseases. For example, as the digital therapy module data, information about the existing AI model used for determination and management of the existing diseases and digital therapy modules having been used for digital therapy solutions may be provided.

A new digital therapy solution may be developed through the database 123 and the digital therapy solution data provider 126 provided through the digital therapy solution generator 120.

The digital therapy solution tester 140 may be implemented to test the generated digital therapy solution. When a specific digital therapy application is developed, a test targeting a plurality of users may be performed. The test result may include performance comparison data through comparison with the existing digital therapy applications. For example, it may be assumed that the existing digital therapy application for insomnia is present and a new digital therapy application for insomnia therapy is present. In this case, the digital therapy solution tester 140 may perform a test on the therapeutic effect of the existing digital therapy application and the new digital therapy application, and deliver the results to the developer of the new digital therapy application based on the test result.

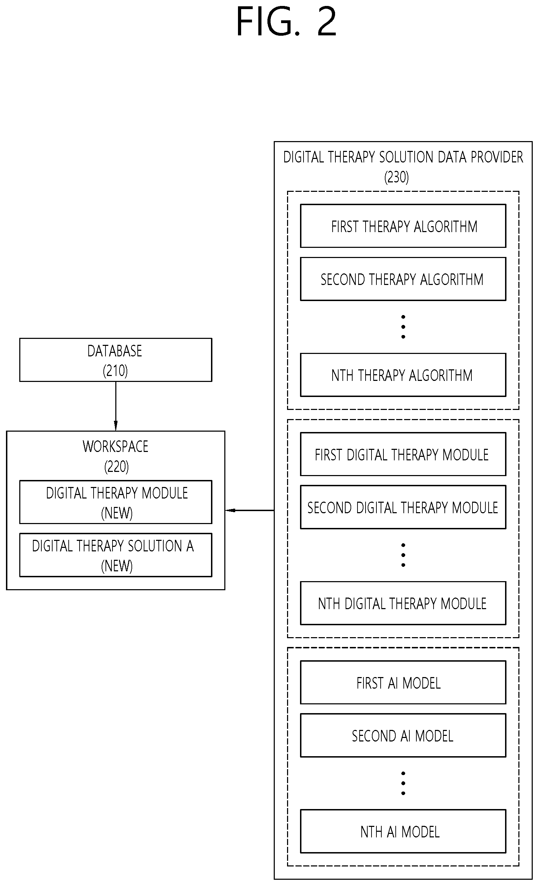

FIG. 2 is a conceptual diagram illustrating the operation of a digital therapy solution generator according to an embodiment of the present invention.

In FIG. 2, an operation of generating a digital therapy solution through a workspace of the digital therapy solution generator is disclosed.

Referring to FIG. 2, a new digital therapy solution and a new digital therapy module may be developed based on data stored in a database 210 by the digital therapy solution generator. For example, it may be assumed that a user, who is a developer, develops a digital therapy application for insomnia therapy.

In order to develop a digital therapy application for insomnia, the existing insomnia patient data (symptom data, therapy data, and the like) may be provided from the database 210.

In addition, digital therapy solution data for the existing insomnia therapy may be provided through the digital therapy solution data provider 230. For example, data regarding a digital therapy module (e.g., an AI module, a sleep time determination module, and the like) and a therapy algorithm (e.g., an insomnia therapy algorithm, an eating disorder therapy algorithm, and a migraine therapy algorithm), which have been used for a digital therapy solution having been used for the existing insomnia therapy, and an AI model (an insomnia therapy AI model, an eating disorder therapy AI model, and a migraine therapy AI model) may be provided through the digital therapy solution data provider 230.

In addition, specific development information, such as source codes, learning data and the like for individual modules (e.g., an AI module, a sleep time determination module, and the like) may be provided through the digital therapy solution data provider 230.

The user may develop a new digital therapy solution and a new digital therapy module based on previous digital therapy history data and digital therapy solution data through a workspace 220.

In addition, according to an embodiment of the present invention, the digital therapy solution generator may provide previous digital therapy history data and digital therapy solution data for developing a digital therapy solution based on keywords or tag information. For example, when a user, who is a developer, desires to develop a digital therapy solution related to migraines, a function of searching with the keyword "migraine" may be provided, and search results for previous digital therapy history data and digital therapy solution data may be provided to the user.

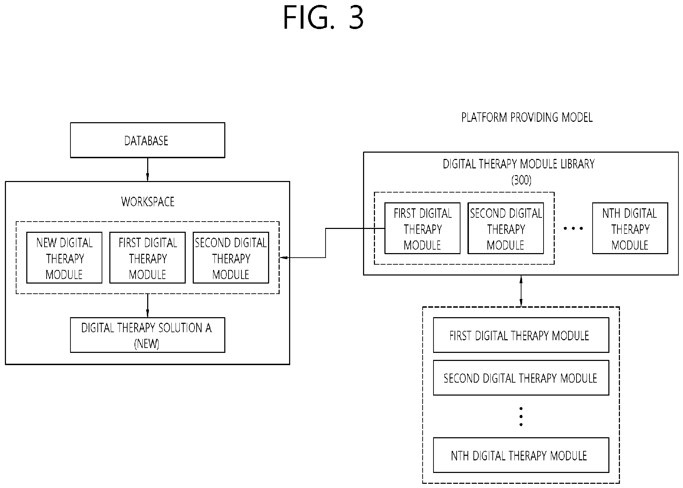

FIG. 3 is a conceptual diagram illustrating the operation of the digital therapy solution generator according to an embodiment of the present invention.

In FIG. 3, an operation of generating a digital therapy solution through a workspace of the digital therapy solution generator is disclosed. In FIG. 3, only a digital therapy module library for digital therapy modules is disclosed, but a therapy algorithm library, an AI model library, a digital therapy data library, and the like may be present.

Referring to FIG. 3, a digital therapy module may be selected through a digital therapy module library 300 included in the digital therapy module data provider. The digital therapy module library 300 may provide information about a digital therapy module used in a digital therapy solution in association with digital therapy solution data in a library format.

Digital therapy modules may be selected from the digital therapy module library 300, and the digital therapy modules may be combined in the workspace. In addition, a digital therapy module newly developed in a user workspace may be combined with the existing digital therapy module such that a new digital therapy solution may be developed.

In addition, according to the performance update of the digital therapy module in the digital therapy module library 300, the existing digital therapy solution related to the updated digital therapy module may be updated.

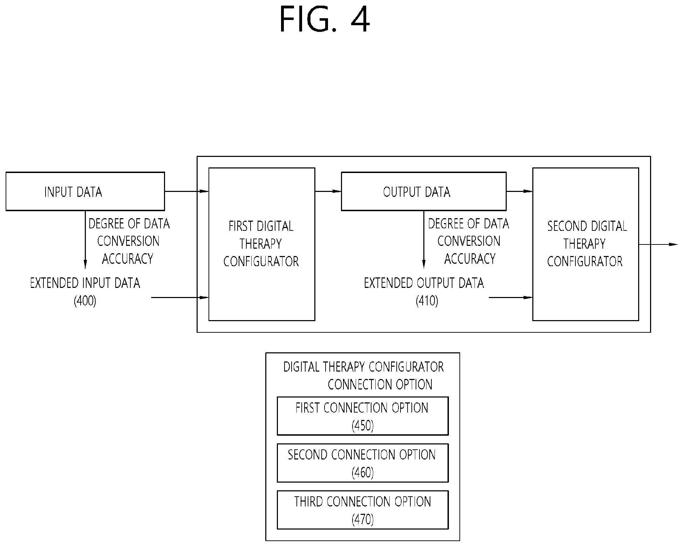

FIG. 4 is a conceptual diagram illustrating a method of developing a digital therapy solution according to an embodiment of the present invention.

In FIG. 4, a method of developing a digital therapy solution in consideration of user input data is disclosed.

Referring to FIG. 4, a method of developing a digital therapy solution in consideration of user input data that may be input by a user is disclosed. Hereinafter, for convenience of description, a module, an AI model, and an algorithm for developing a digital therapy solution, such as the digital therapy module, the AI model, and the algorithm, may be expressed by the term "digital therapy configurators."

Each of the digital therapy configurators may include input information and output information, and a connection relationship between the digital therapy configurators may be established through matching between the input information and the output information.

Settings may be performed on a digital therapy configurator that receives the initial data based on user input data that may be input. Essential input information may be set in the digital therapy configurator, and it may be determined whether user input data includes the essential input information of the initial digital therapy configurator that receives the user input data.

Input data and output data may be set as extended input data 400 and extended output data 410 in consideration of whether conversion between data is possible and whether substitution of data is possible, even when the input data and the output data do not have the same format.

In order to set the extended input data 400 and the extended output data 410, the possibility of conversion between input data and other input data and the possibility of conversion between input data and other input data may be identified.

The possibility of conversion between data (input data/output data) may be determined by considering the degree of data conversion accuracy during converting of data, which is based on a relationship between data. The degree of data conversion accuracy may be determined by considering the relationship between data such as when a specific mathematical relationship is present or a relationship between two pieces of data is already established in a database based on existing data. The degree of data conversion accuracy may be determined by considering an error probability and an error range during data conversion.

When the degree of data conversion accuracy is greater than or equal to a threshold value, input data may be set as extended data for the input data.

First connection option 450: The first connection option 350 may be set as a digital therapy configurator that is connectable only when input data and output data match each other.

Second connection option 460: The second connection option 460 may be set as a digital therapy configurator that is connectable when connection is allowable through the setting of extended input data/extended output data even when the input data and the output data do not match each other.

Third connection option 470: The third connection option 470 may be set for a digital therapy configurator to be connected through a coupling of a partial first connection option (a partial first connection option) and a partial second connection option (a partial second connection option).

Among a plurality of digital therapy configurators constituting a digital therapy solution, a part set as a core digital therapy configurator having relatively high importance may be connected with a partial first connection option 450, and a part set as a non-core digital therapy configurator having relatively low importance may be connected with a partial second connection option 460.

In addition, according to an embodiment of the present invention, the possibility of conversion between data (input data/output data) may be determined based on auxiliary data. For example, it may be assumed that conversion of first input data into second input data is required. Even in a case in which the first input data with auxiliary data added thereto is converted into second input data, when the degree of data conversion accuracy is higher than a threshold value, a digital therapy configurator may be recommended based on the possibility of securing the auxiliary data.

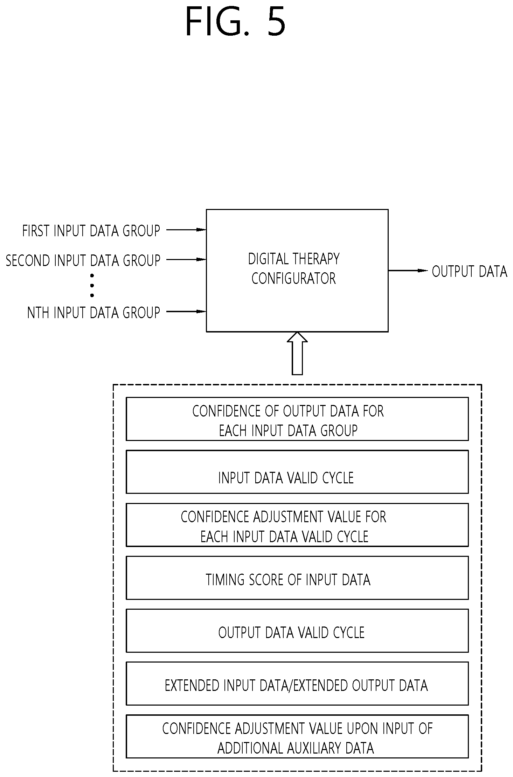

FIG. 5 is a conceptual diagram illustrating a method of standardizing digital therapy configurators for connection of digital therapy configurators according to an embodiment of the present invention.

In FIG. 5, a method of standardizing and defining digital therapy configurators for connection between the digital therapy configurators is disclosed.

Referring to FIG. 5, for a digital therapy configurator, input data and output data may be defined and set in consideration of characteristics of the digital therapy configurator.

Input data of a digital therapy configurator may be defined based on a definition of input data or an input data group that may be input to the digital therapy configurator and information about the confidence of output data upon an input according to the input data or the input data group. Hereinafter, for convenience of description, a group including at least one piece of input data input to a digital therapy configurator is defined as an input data group.

For example, the digital therapy configurator is a digital therapy module, and various data groups capable of generating output data may be set for the digital therapy module. For example, the input data groups may be set as a first input data group, a second input data group, and a third input data group, and the confidences of output data according to each of the first input data group, the second input data group, and the third input data group may be set.

In addition, a generation time of input data included in an input data group may be set for the digital therapy configurator. For example, with respect to first input data to n^{th} input data included in an input data group, an input data valid cycle for each of the pieces of input data that are capable of forming a single input data group as a plurality of pieces of input data may be defined. Input data included in an input data valid cycle for each piece of input data may form a single input data group. As the input data changes relatively rapidly, the input data valid cycle may be defined to be shorter.

For each digital therapy configurator, a confidence adjustment value for each input data cycle may be set such that, when using input data deviating from an input data valid cycle of input data, the confidence of output data is adjusted in consideration of the degree (e.g., a deviation of one cycle) (hereinafter referred to as a cycle-specific validity score). The valid time cycles may be defined to be different for each piece of input data, and the cycle-specific validity scores may be set to be different for each piece of input data, so that the confidence of the output data may be adjusted.

In addition, based on proximity of the generation times of a plurality of pieces of input data within an input data valid cycle, a timing score of input data may be determined, and a confidence adjustment value of output data may be defined according to the timing score of input data. A higher concurrency of input data results in a higher timing score of the input data, and in response to an increase in the timing score of the input data, the confidence of the output data may be adjusted to be higher. Timing-center input data for determining the timing score may be determined, and the timing-center input data may be input data that most influences the confidence of output data.

In addition, with respect to output data of the digital therapy configurator, an output data valid cycle may be defined. The output data valid cycle may be defined based on the input data valid cycle, but as the output data changes relatively rapidly, the output data valid cycle may be defined to be shorter.

There may be cases in which output data of a digital therapy configurator is used as input data of another digital therapy configurator, and such output data may be defined using the term "input data (output)". An input data valid cycle of the input data (output) used as the input data may be determined based on the output data valid cycle. In this case, the input data valid cycle of the input data (output) input to the other digital therapy configurator may be adjusted based on input data valid cycles of other input data input to the other digital therapy configurator.

When the average value of the input data valid cycles of the other input data is shorter than the input data valid cycle of the input data (output), the input data valid cycle of the input data (output) may be adjusted to be shorter based on the average value of the input data valid cycles of the other input data. Conversely, when the average value of the input data valid cycles of the other input data is longer than the input data valid cycle of the input data (output), the input data valid cycle of the input data (output) may be adjusted to be longer based on the average value of the input data valid cycles of the other input data. Through this method, the time difference between input data is adjusted, thereby minimizing the effect on the confidence of output data.

As described above, extended input data and extended output data of the digital therapy configurator may be defined, and the extended input data and the extended output data may be changed according to changes in the relationship between data. In addition, auxiliary data other than input data may be defined, and settings for a change in confidence upon additional input of auxiliary data may also be defined.

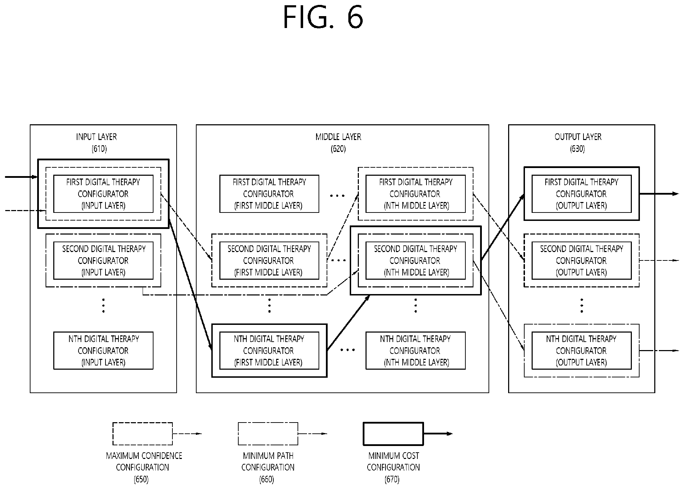

FIG. 6 is a conceptual diagram illustrating a connection relationship between digital therapy configurators according to an embodiment of the present invention.

In FIG. 6, a connection algorithm for connecting digital therapy configurators to implement a digital therapy solution is disclosed.

Referring to FIG. 6, a user who generates a digital therapy solution in a workspace for connection of digital therapy configurators may select available input data and input information about desired output data.

In this case, a target digital therapy configurator that is connectable may be recommended based on the input data and the output data.

For the recommendation of the target digital therapy configurator, the digital therapy configurator may be defined for each layer. The layer may include an input layer 610, a middle layer 620 (a first middle layer to an n^{th} middle layer), and an output layer 630. The input layer 610 may be a layer that receives input data, the middle layer 620 may be a layer used to connect input data and output data (or an input layer and an output layer), and the output layer 630 may be a layer that outputs output data.

In the present invention, the target digital therapy configurator may be recommended based on different configurations, such as a maximum confidence configuration 650, a minimum path configuration 660, and a minimum cost configuration 670.

The maximum confidence configuration 650 may be based on a connection between target digital therapy configurators that provides output data with the highest confidence. A combination of digital therapy configurators capable of maximizing the confidence of output data based on various combinations of digital therapy configurators may be included in the maximum confidence configuration 650.

The minimum path configuration 660 may be a configuration using the fewest number of targeted digital therapy configurators.

The minimum cost configuration 670 may be a configuration for outputting output data at the lowest cost. The cost may be calculated based on various costs, such as the cost (e.g., license fees) of using digital therapy configurators, the cost of using computing resources used to use digital therapy configurators, and the like.

In the present invention, such various configurations of a digital therapy configurator are recommended, and the user may alternatively select a digital therapy configurator to generate a digital therapy solution.

In addition, in the embodiment of the present invention, optionally, an extended maximum confidence configuration, an extended minimum path configuration, and an extended minimum cost configuration extended for the maximum confidence configuration 650, the minimum path configuration 660, and the minimum cost configuration 670 may be newly defined through selection of extended input data and extended output data, and a target digital therapy configurator may be newly set accordingly.

Alternatively, more specifically, the user may additionally input information about the valid cycle of available input data, the input possibility of additional auxiliary data, and the confidence of input data, based on which a recommendation for a target digital therapy configurator may be performed.

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the spirit and scope of the present invention are defined not by the detailed description of the present invention but by the appended claims, and encompass all modifications and equivalents that fall within the scope of the appended claims.

## Claims

1. A method of developing a therapy solution through a modular digital therapy framework, the method comprising:
receiving, by a digital therapy solution generator, digital therapy solution data; and
generating, by the digital therapy solution generator, a digital therapy solution based on the digital therapy solution data.

2. The method of claim 1, wherein the digital therapy solution data includes data regarding a digital therapy module, a therapy algorithm, or an artificial intelligence (AI) model.

3. The method of claim 2, wherein the digital therapy solution is generated based on a combination of the digital therapy module, the therapy algorithm, or the AI model.

4. A system for generating a digital therapy solution, which is a system for developing a digital therapy solution through a modular digital therapy framework, the system comprising a digital therapy solution generator configured to receive digital therapy solution data and generate a digital therapy solution based on the digital therapy solution data.

5. The system of claim 4, wherein the digital therapy solution data includes data regarding a digital therapy module, a therapy algorithm, or an artificial intelligence (AI) model.

6. The system of claim 5, wherein the digital therapy solution is generated based on a combination of the digital therapy module, the therapy algorithm, or the AI model.
